# EUROPÄISCHE PATENTSCHRIFT

(11) **EP 3 416 597 B1**
(45) Veröffentlichungstag und Bekanntmachung des Hinweises auf die Patenterteilung: **12.08.2020**
(21) Anmeldenummer: 17704014.4
(22) Anmeldetag: 09.02.2017
(51) Int. Cl.: A61F 5/01, A61F 13/00

(54) **UMLENKELEMENT ZUM UMLENKEN VON SEILEN IN ODER AN ORTHOPÄDISCHEN ODER MEDIZINISCHEN HILFSMITTELN ODER SPORTHILFSMITTELN**
DIVERTING ELEMENT FOR DIVERTING ROPES IN OR AT ORTHOPÄDIC OR MEDICAL OR SPORT AIDS
ÉLÉMENT DE DÉVIATION POUR LA DÉVIATION DE CORDES EN OUTILS OTHOPÉDIQUES, MEDICALES OU DE SPORT

(30) Priorität: 16.02.2016 DE 202016100799 U; 16.02.2016 US 201662295830 P
(43) Veröffentlichungstag der Anmeldung: 26.12.2018
(73) Patentinhaber: Bauerfeind AG, 07937 Zeulenroda-Triebes (DE)
(72) Erfinder: BECK, André, 07646 Stadtroda (DE)
(74) Vertreter: Wohlfahrt, Jan Günther
(86) Internationale Anmeldenummer: PCT/EP2017/052918
(87) Internationale Veröffentlichungsnummer: WO 2017/140578

(56) Entgegenhaltungen:
- WO-A1-2010/098880
- WO-A1-2012/109524
- US-A1- 2002 074 373
- US-A1- 2012 215 254
- US-A1- 2013 237 891
- US-A1- 2013 237 891
- US-B1- 6 322 529

## Beschreibung

Die vorliegende Erfindung betrifft Elemente zum Umlenken von Seilen, insbesondere Schnüren, insbesondere Spannseilen oder Spannschnüren in oder an orthopädischen oder medizinischen Hilfsmitteln oder Sporthilfsmitteln, insbesondere in oder an Orthesen oder Bandagen. Die US 6 322 529 B1 stellt den nächsten Stand der Technik dar.

In orthopädischen oder medizinischen Hilfsmitteln beziehungsweise Sporthilfsmitteln werden häufig Gurte und Bänder als Zuggurte oder Spanngurte eingesetzt. Beispielsweise beschreibt die DE 10 2010 054 579 A1 eine Orthese für die Dämpfung oder Begrenzung der Gelenkbewegung eines Extremitätengelenks, bei der eine Manschette und ein Widerlager zu der Manschette über mindestens ein Zugband kraftschlüssig verbunden sind.

Jedoch sollen solche orthopädischen Hilfsmittel, medizinischen Hilfsmittel oder Sporthilfsmittel, insbesondere Orthesen oder Bandagen zukünftig immer schlanker, leichter und dünner werden, wobei die Wirksamkeit mindestens gleich bleiben soll. Um dies zu gewährleisten wäre es vorteilhaft, bislang verwendete Zuggurte oder Zugbänder durch Seile, insbesondere dünne Schnüre, also Seile mit einem geringen Durchmesser zu ersetzen, beispielsweise eine Gelenkbewegung durch ein Schnursystem einer solchen Orthese oder Bandage zu führen.

Auch haben Seile und insbesondere Schnüre gegenüber Bändern und Gurten den Vorteil, dass sie durch ihren dünnen und runden Querschnitt weniger Reibung auf dem sie umgebenden Material verursachen.

Bei der Verwendung von Seilen, insbesondere dünnen Schnüren zur Krafteinleitung kommt es allerdings dazu, dass das Seil, insbesondere die Schnur, bedingt durch den geringen Querschnitt in die Haut des Trägers einschneiden kann und somit der Tragekomfort verringert wird.

Darüber hinaus stellt sich das Problem, dass insbesondere eine Orthese in ihrer Position sicher am Körper sitzen muss, um ihre Wirkung bestmöglich entfalten zu können. Dies wird im Stand der Technik durch das Spannen von Gurten erreicht. Bei dem Spannvorgang kann es jedoch vorkommen, dass durch das Zusammenziehen der Gurte die Haut des Trägers verzogen oder gar eingeklemmt wird.

Der vorliegenden Erfindung liegt daher das technische Problem zugrunde, die Nachteile aus dem Stand der Technik zu verbessern. Insbesondere sollen Vorrichtungen und Elemente bereitgestellt werden, die es erlauben, die in orthopädischen oder medizinischen Hilfsmitteln oder Sporthilfsmitteln, insbesondere Orthesen oder Bandagen, eingesetzten Bänder und Gurte, insbesondere Spann- oder Zuggurte durch Seile, insbesondere dünne Schnüre, ersetzen zu können, wobei dabei insbesondere die Kraft der Seile so verteilt wird, dass die Seile nicht in die Haut des Trägers einschneiden. Ein weiteres Problem, das der vorliegenden Erfindung zugrunde liegt, ist die Breitstellung einer Vorrichtung, die bei einer Umfangsveränderung eines oben genannten Hilfsmittels nicht zu einem Verschieben oder Einklemmen des darunter liegenden Hautgewebes führt.

Die vorliegende Erfindung löst das ihr zugrunde liegende technische Problem insbesondere durch ein Umlenkelement nach Anspruch 1 und durch ein orthopädisches Hilfsmittel, medizinisches Hilfsmittel oder Sporthilfsmittel nach Anspruch 10.

Die vorliegende Erfindung betrifft insbesondere ein Umlenkelement zum Umlenken eines Seils in orthopädischen oder medizinischen Hilfsmitteln oder Sporthilfsmitteln, wobei das Umlenkelement wenigstens ein erstes Teilelement und ein zweites Teilelement umfasst, wobei das erste Teilelement und das zweite Teilelement mit nur jeweils einem Freiheitsgrad gegeneinander verschiebbar sind, und wobei das erste Teilelement und das zweite Teilelement jeweils mindestens ein Seilführungselement zum Umlenken des Seils aufweisen.

Bevorzugt bestehen die Teilelemente aus einem formstabilen Material, insbesondere aus Kunststoff oder aus Metall. Bevorzugt wird für die Schalen ein Material mit geringen Reibungseigenschaften gewählt.

Bevorzugt ist das Umlenkelement in oder an einem orthopädischen oder medizinischen Hilfsmittel oder Sporthilfsmittel, insbesondere in oder an einer Orthese oder Bandage befestigt, insbesondere Bestandteil eines orthopädischen oder medizinischen Hilfsmittels oder Sporthilfsmittels, insbesondere einer Orthese oder Bandage.

Es zeigte sich, dass solche Umlenkelemente aus zwei Teilelementen, die gegeneinander verschiebbar gelagert sind, insbesondere schalenförmigen Teilelementen, und die darüber hinaus jeweils mindestens ein, insbesondere zwei Seilführungselemente zum Umlenken eines Seils, insbesondere einer Schnur aufweisen, es ermöglichen, bei der Verwendung eines Seils, insbesondere einer Schnur in einem medizinischen Hilfsmittel, orthopädischen Hilfsmittel oder Sporthilfsmittel, insbesondere in einer Orthese oder Bandage, dort verwendete Zuggurte oder Spanngurte durch Seile, insbesondere Schnüre zu ersetzen und eine Umlenkung dieser Seile, insbesondere Schnüre möglich ist, ohne dass die Seile, insbesondere Schnüre in die Haut des Trägers des Hilfsmittels einschneiden. Dies wird insbesondere auch dadurch erreicht, dass die Teilelemente in ihrer bevorzugten Schalenform großflächig die Haut des Trägers abdecken und im Bereich der Umlenkung der Seile, insbesondere Schnüre, ein Einschneiden in die Haut durch die großflächige Kraftverteilung verhindern.

Das erfindungsgemäße Umlenkelement mit seinen gegeneinander verschiebbaren Teilelementen hat darüber hinaus den technischen Vorteil, dass das Seil über die Seilführungselemente der zwei Teilelemente geführt werden kann, sodass beim Auseinanderschieben der beiden Teilelemente gegeneinander der Seilverlauf in dem Umlenkelement verlängert wird und somit ein Spannen des Seils erreicht wird. Darüber hinaus gleiten die beiden Teilelemente gegeneinander, sodass die unter dem Umlenkelement liegende Haut des Trägers nicht verschoben oder eingeklemmt wird.

In einer bevorzugten Ausführungsform weist das Umlenkelement eine Fixiereinrichtung auf, durch die der jeweils eine Freiheitsgrad blockiert werden kann. In einer bevorzugten Ausführungsform ist die Fixiereinrichtung ein Steckelement, das zwischen das erste Teilelement und das zweite Teilelement gesteckt wird.

Eine solche Fixiereinrichtung hat den Vorteil, dass beim Fixieren der Fixiervorrichtung, beispielsweise beim Einstecken des Steckelements zwischen das erste und das zweite Teilelement diese sich nicht mehr zusammen bewegen können, sodass das in dem Umlenkelement befindliche Seil gespannt bleibt.

In einer bevorzugten Ausführungsform weist das erste Teilelement und/oder das zweite Teilelement mindestens zwei randseitige Führungen auf, in denen das jeweils andere Teilelement gleiten kann. In einer bevorzugten Ausführungsform sind die Führungen als Nutführungen ausgestaltet.

Randseitige Führungen, insbesondere Nutführungen erlauben in vorteilhafter Weise, dass die beiden Teilelemente gegeneinander mit nur einem Freiheitsgrad durch Gleiten verschoben werden können, aber gleichzeitig miteinander fixiert sind, und ein Verschieben in andere Freiheitsgrade verhindert wird.

In einer bevorzugten Ausführungsform haben die beiden Teilelemente jeweils einen plattenförmigen Grundkörper, aus dem auf einer Seite das mindestens eine Seilführungselement und die mindestens zwei randseitigen Führungen herausragen.

Ein plattenförmiger Grundkörper der beiden Teilelemente führt zu einer vorteilhaften großflächigen Schalenform. Die beiden Platten werden durch die randseitigen Führungen mit etwas Abstand zusammengehalten, sodass sich zwischen den beiden Platten die Seilführungselemente zum Umlenken des Seils befinden können und das Seil somit zwischen den beiden Platten geführt werden kann.

In einer bevorzugten Ausführungsform bilden die beiden Teilelemente einen Innenraum, dessen Größe durch das Verschieben der beiden Teilelemente gegeneinander veränderbar ist, wobei die Seilführungselemente in den Innenraum hineinragen.

In einer bevorzugten Ausführungsform findet die Verschiebung der beiden Teilelemente zueinander in einem Kreisbogen statt und durch die Verschiebung wird die Bogenlänge eines Kreissegmentes verändert.

In dieser Ausführungsform eignet sich das Umlenkelement insbesondere bei der Verwendung eines orthopädischen oder medizinischen Hilfsmittels im Bereich einer Extremität, aber auch im Bereich des Oberkörpers. Der Radius des Kreisbogens kann in vorteilhafter Weise dem Radius besagter Extremität oder des Oberkörpers angepasst sein. Somit kann dann das Umlenkelement in vorteilhafter Weise die Extremität oder den Oberkörper teilweise umgreifen. Bei einem Auseinanderschieben der beiden Teilelemente wird der umgriffene Anteil dementsprechend vergrößert.

In einer bevorzugten Ausführungsform weist das erste Teilelement und/oder das zweite Teilelement mindestens ein Loch zum Durchführen des Seils auf.

In einer bevorzugten Ausführungsform umfasst das Umlenkelement ein Seil, insbesondere eine Schnur. In einer bevorzugten Ausführungsform ist das Seil eine Schnur, also ein Seil mit einem dünnen Durchmesser. Dem Fachmann sind geeignete Schnüre und geeignete Materialen für solche Schnüre bekannt.

In einer bevorzugten Ausführungsform weisen das erste Teilelement und das zweite Teilelement jeweils zwei Seilführungselemente zum Umlenken des Seils auf, wobei das Seil erst durch das erste Seilführungselement des ersten Teilelements umgelenkt wird, dann durch das erste Seilführungselement des zweiten Teilelements umgelenkt wird, dann durch das zweite Seilführungselement des ersten Teilelements umgelenkt wird und dann durch das zweite Seilführungselement des zweiten Teilelements umgelenkt wird.

Es entsteht somit ein Seilzugsystem, bei dem durch Auseinanderschieben der Teilelemente eine stärkere Spannung des Seils hervorgerufen werden kann. In vorteilhafter Weise kann durch die Anzahl der Seilführungselemente bestimmt werden, welche Seillänge in das Umlenkelement beim Auseinanderziehen der Teilelemente eingezogen wird, und somit, wie stark das Seil angespannt wird.

In einer bevorzugten Ausführungsform führt ein Verschieben der beiden Teilelemente gegeneinander zum Spannen des Seils.

In einer bevorzugten Ausführungsform führt ein Spannen des Seils zum Verschieben der beiden Teilelemente gegeneinander.

Es kann also sowohl vorgesehen sein, dass ein Spannen des Seils in einem anderen Bereich des Hilfsmittels zum Zusammenschieben der Teilelemente führt. Dies kann beispielsweise bei einer bewegungslimitierenden Orthese eingesetzt werden, wenn die Teilelemente gekrümmte seitliche Bereiche aufweisen, die beim Zusammenschieben der Teilelemente herausragen und eine Extremität somit stärker umfassen.

Anders herum kann wie beschrieben auch ein Auseinanderziehen der Teilelemente zum Spannen eines Seils genutzt werden, wobei dann die Teilelemente durch eine Fixiereinrichtung in der auseinander geschobenen Position fixiert werden können, sodass das Seil gespannt bleibt.

In einer bevorzugten Ausführungsform tritt das Seil in das Umlenkelement ein und nach der Umlenkung wieder aus dem Umlenkelement aus.

In einer alternativen Ausführungsform tritt das Seil in das Umlenkelement ein und ist nach der Umlenkung mit einem Ende am Umlenkelement befestigt.

Es kann also sowohl vorgesehen sein, dass das Seil von einem anderen Bereich eines Hilfsmittels zu dem Umlenkelement geführt wird, in das Umlenkelement hineinführt, dort umgelenkt wird und dann wieder aus dem Umlenkelement herausgeführt wird und zu einem weiteren Bereich des Hilfsmittels geführt wird. Alternativ kann es auch vorgesehen sein, dass das Seil an einem Teilelement des Umlenkelements befestigt ist, dann durch das Umlenkelement umgelenkt wird, und nur ein einfach aus dem Umlenkelement heraustritt und zu einem weiteren Bereich des Hilfsmittels geführt wird.

Die vorliegende Erfindung betrifft auch ein orthopädisches Hilfsmittel, ein medizinisches Hilfsmittel oder ein Sporthilfsmittel, umfassend ein erfindungsgemäßes Umlenkelement.

In einer bevorzugten Ausführungsform weist das orthopädische Hilfsmittel, das medizinische Hilfsmittel oder das Sporthilfsmittel ein Spannseil auf, dem das Umlenkelement zugeordnet ist.

In einer bevorzugten Ausführungsform ist das orthopädische Hilfsmittel eine Orthese.

Bei der Orthese kann es sich beispielsweise um eine Armorthese, Beinorthese oder Rückenorthese handeln. Bevorzugt handelt es sich um eine Orthese für Extremitäten. Beispielsweise kann es sich um eine Orthese handeln, die eine stützende oder bewegungsgleitende Funktion ausübt, insbesondere eine Orthese zur Dämpfung oder Begrenzung der Beugung oder Streckbewegung eines Extremitätengelenks, wie Ellenbogengelenk oder Kniegelenk. Eine solche Orthese ist beispielsweise aus der DE 10 2010 054 579 A1 bekannt. Ein Fachmann kann ohne weiteres die dort gezeigten Zugbänder durch Seile, insbesondere Schnüre und den dort gezeigten Druckeinleitungsabschnitt durch ein erfindungsgemäßes Umlenkelement ersetzen. Die vorliegende Erfindung betrifft daher bevorzugt auch eine dementsprechend modifizierte Orthese, wie sie in der DE 10 2010 054 579 A1 beschrieben ist. Der Gegenstand der DE 10 2010 054 579 A1 ist daher in die vorliegende Offenbarung mit einbezogen.

In einer bevorzugten Ausführungsform ist die Orthese eine Orthese zur Dämpfung oder Begrenzung der Gelenkbewegung eines Extremitätengelenks, aufweisend eine die Extremität unterhalb des Gelenks umgreifende Manschette, die mit einem an der Extremität oberhalb des Gelenks anlegbaren Widerlager gekoppelt ist, wobei sich von dem Widerlager zu der Manschette mindestens ein Zugseil erstreckt, das mit Widerlager und Manschette kraftschlüssig verbunden ist, wobei das Zugseil über ein erfindungsgemäßes Umlenkelement mit der Manschette verbunden ist und das Umlenkelement als Druckeinleitungsabschnitt der Manschette ausgebildet ist, und wobei das Zugseil derart ausgestaltet ist, dass es im angelegten Zustand der Orthese durch die Gelenkbewegung der Extremität spannbar ist, um dabei über das Umlenkelement Kompression auf einen darunter liegenden Weichteilbereich der Extremität auszuüben, um die Gelenkbewegung zu dämpfen oder zu begrenzen.

In einer bevorzugten Ausführungsform verläuft bei der Orthese das Zugseil zweifach und überkreuzt von dem Widerlager zu dem Druckeinleitungsabschnitt der Manschette und wird an dem Druckeinleitungsabschnitt durch das erfindungsgemäße Umlenkelement umgelenkt.

In einer bevorzugten Ausführungsform ist das Widerlager als zweite Manschette ausgebildet.

In einer bevorzugten Ausführungsform handelt es sich bei der ersten Manschette und/oder bei der zweiten Manschette um flexible Manschettengurte, die bevorzugt mittels Klettverschluss geöffnet und angezogen werden können.

In einer bevorzugten Ausführungsform sind Manschette und Widerlager auf ein textiles Gestrick aufgerüstet.

In einer bevorzugten Ausführungsform kann das Zugseil im Bereich des Widerlagers durch ein Aufrollelement gespannt werden. Es wird also in dieser bevorzugten Ausführungsform das Zugseil durch das Aufrollelement gespannt, wodurch im Bereich der ersten Manschette die beiden Teilelemente des Umlenkelements zusammen geschoben werden, und wobei bevorzugt die Verschiebung der beiden Teilelemente zueinander in einem Kreisbogen stattfindet und das Umlenkelement die Extremität stärker umgreift.

Bevorzugte Ausführungsformen ergeben sich auch aus den Unteransprüchen.

Die vorliegende Erfindung wird anhand der Figuren näher und beispielhaft erläutert, wobei die Figuren und dazugehörige Erläuterung nicht einschränkend zu verstehen sind.

Es zeigen
- Figur 1: ein erfindungsgemäßes Umlenkelement mit eingeschobenem Steckelement;
- Figur 2: das erfindungsgemäße Umlenkelement von Figur 1 mit herausgezogenem Steckelement;
- Figur 3: das erfindungsgemäße Umlenkelement von Figur 2 mit zusammengeschobenen Teilelementen;
- Figur 4: ein erfindungsgemäßes Umlenkelement mit einer alternativen Umlenkung der Schnur;
- Figur 5: ein erfindungsgemäßes Umlenkelement mit einer weiteren alternativen Umlenkung der Schnur;
- Figur 6: ein erfindungsgemäßes Umlenkelement mit einer weiteren alternativen Umlenkung der Schnur;
- Figur 7: die zwei Teilelemente eines erfindungsgemäßen Umlenkelements;
- Figur 8: eine weitere Ansicht eines erfindungsgemäßen Umlenkelements;
- Figur 9: die zwei Teilelemente eines erfindungsgemäßen Umlenkelements;
- Figur 10: eine erfindungsgemäße Orthese zur Dämpfung oder Begrenzung der Gelenkbewegung eines Ellenbogens;
- Figur 11: eine weitere Ansicht der Orthese aus Figur 10;
- Figur 12: eine weitere Ansicht der Orthese aus Figur 10;
- Figur 13: das Umlenkelement aus Figur 8 mit an den Teilelementen angebrachtem Gurt;
- Figur 14: das Umlenkelement aus Figur 8 mit an den Teilelementen alternativ angebrachtem Gurt.

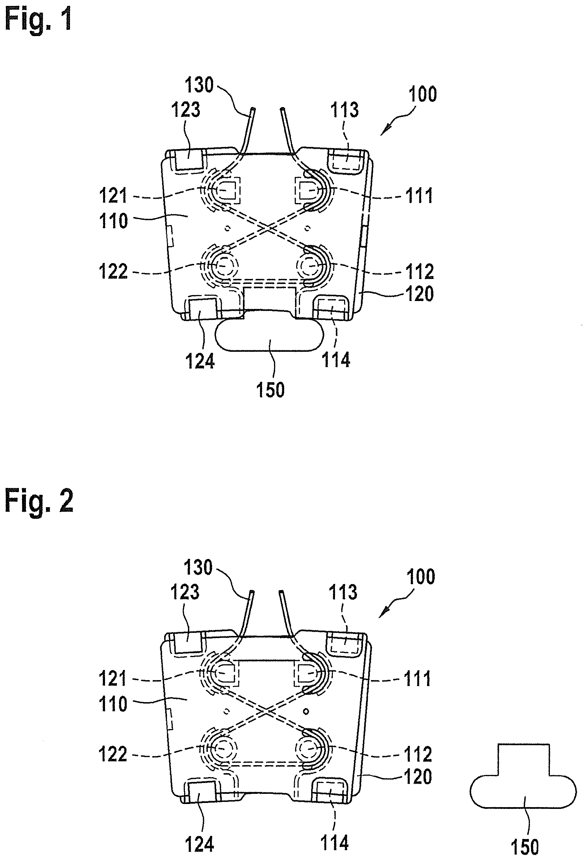

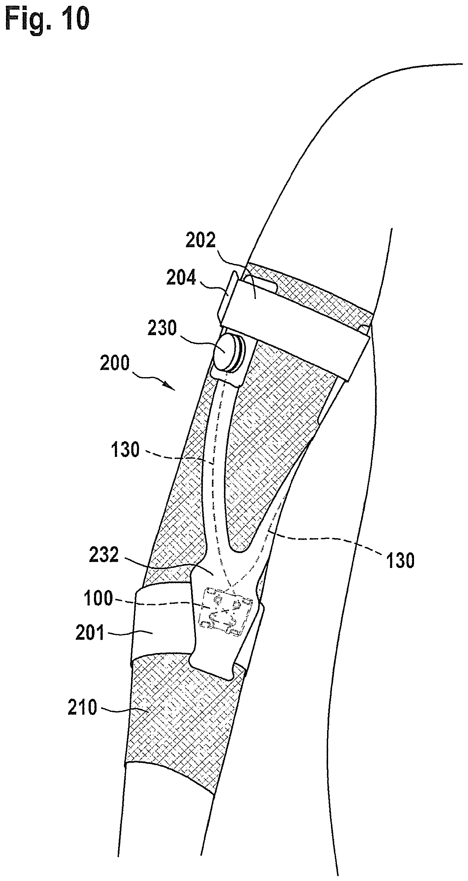

Gezeigt sind in den Figuren 1 bis 9 zwei formstabile ineinanderlaufende Teilelemente (110,120) in Form von Schalenelementen. Die Schalenelemente können flach ausgestaltet sein oder als Zylinderausschnitt oder als Kegelausschnitt anatomisch angeformt sein. Jedes Schalenelement weist zwei Seilführungselemente (111,112,121,122) zur Schnurführung auf. Eine krafteinleitende Schnur (130) läuft im Zwischenraum der Schalenelemente. Die Schnur kann zwischen den Schalen ringförmig, parallel, überkreuz oder in einer Art Flaschenzug verlaufen. Neben einer linearen Krafteinleitung kann durch die Schalen die Spannkraft um 10° bis 170°, bevorzugt um etwa 90° umgelenkt werden. Die Spannschalen können ihrerseits an einer Bandage oder Orthese (200) kraftschlüssig befestigt werden, wie sie beispielsweise in den Figuren 10 bis 12 gezeigt ist. Bei Längenänderung der Schnur (130) werden die Schalenelemente (11,120) aufeinander gezogen und die Bandage oder Orthese (200) wird an den Körper herangezogen. Da die Schalen ein geschlossenes System bilden, gibt es keine Zwischenräume, in denen beim Spannen Haut eingeklemmt werden oder die Schnur einschneiden kann. Bei Knieorthesen können die Spannschalen beispielsweise gleichzeitig den Ortheserahmen bilden. Die Spannschalentechnik ermöglicht es Bewegungen dadurch zu dämpfen, dass die Schalen zusammengeschoben werden und die eigene Muskulatur als Dämpfer wirkt. Therapiebegleitend kann das Zusammenschieben der Elemente unter Zuhilfenahme einer Fixiereinrichtung, beispielsweise eines Steckelements (150) in der Art eines Splintes zu Beginn der Therapie unterbunden werden. Im weiteren Therapieverlauf wird dieser Splint wieder entfernt und die Dämpfungseffekte können wieder genutzt werden.

Länge und Breite des Verstellweges und somit Dämpfungsweges sind durch Skalierung der Spannschalen (110,120) an die jeweiligen produktspezifischen Anforderungen der Orthesen oder Bandagen anpassbar.

Figur 1 zeigt ein erfindungsgemäßes Umlenkelement (100) mit eingeschobenem Steckelement (150). Das Umlenkelement (100) dient zum Umlenken einer Schnur (130) in orthopädischen oder medizinischen Hilfsmitteln oder Sporthilfsmitteln, beispielsweise der in den Figuren 10 bis 12 gezeigten Orthese zur Dämpfung oder Begrenzung der Gelenkbewegung eines Ellenbogens. Das Umlenkelement (100) umfasst ein erstes Teilelement (110) und ein zweites Teilelement (120), wobei das erste Teilelement (110) und das zweite Teilelement (120) jeweils einen plattenförmigen Grundkörper haben und mit nur jeweils einem Freiheitsgrad gegeneinander verschiebbar sind. Das erste Teilelement (110) weist zwei Seilführungselemente (111, 112) auf und das zweite Teilelement weist zwei Seilführungselemente (121, 122) auf. Die vier Seilführungselemente (111, 112, 121, 122) dienen zum Umlenken der Schnur (130). Das erste Teilelement (110) weist zwei randseitige Führungen (113, 114) auf, die als Nutführungen das zweite Teilelement (120) umgreifen. Auch das zweite Teilelement (120) weist zwei randseitige Führungen (123, 124) auf, die als Nutführungen das erste Teilelement (110) umgreifen. Somit kann ein Teilelement jeweils in den randseitigen Führungen des anderen Teilelements gleiten.

Die beiden Teilelemente (110, 120) bilden einen Innenraum, dessen Größe durch das Verschieben der beiden Teilelemente gegeneinander veränderbar ist: die Seilführungselemente (111, 112, 121, 122) ragen in den Innenraum hinein.

Die beiden Teilelemente (110, 120) sind in Figur 1 in auseinandergeschobener Position gezeigt. Daher liegen die beiden Seilführungselemente (111, 112) des ersten Teilelements (110) in größtmöglicher Entfernung von den beiden Seilführungselementen (121, 122) des zweiten Teilelements (110), so dass die Schnur (130), die abwechselnd von einem Seilführungselement des ersten Teilelements und von einem Seilführungselement des zweiten Teilelements umgelenkt wird auf einem weiten Weg in dem Umlenkelement (100) verläuft, so dass dabei viel Schnur verbraucht wird und die Schnur, wenn sie an ihren nicht gezeigten Enden an einem Widerlager befestigt ist, gespannt ist. Damit diese Spannung aufrechterhalten wird, ist eine als Steckelement (150) ausgebildete Fixiereinrichtung zwischen die beiden Teilelemente (110, 120) eingeschoben, so dass diese nicht zusammengeschoben werden oder sich zusammenziehen können und der eine Freiheitsgrad somit blockiert ist.

In Figur 2 ist das Steckelement (150) herausgezogen, so dass die beiden Teilelemente (110, 120) zwar immer noch in der auseinandergeschobenen Position sind, aber entlang des einen Freiheitsgrads zusammengeschoben werden können, beispielsweise durch über die Schnur (130) wirkende Zugkräfte. Zu sehen sind auch wieder die vier Seilführungselemente (111, 112, 121, 122) und die vier randseitigen Führungen (113, 114, 123, 124) des Umlenkelements (100).

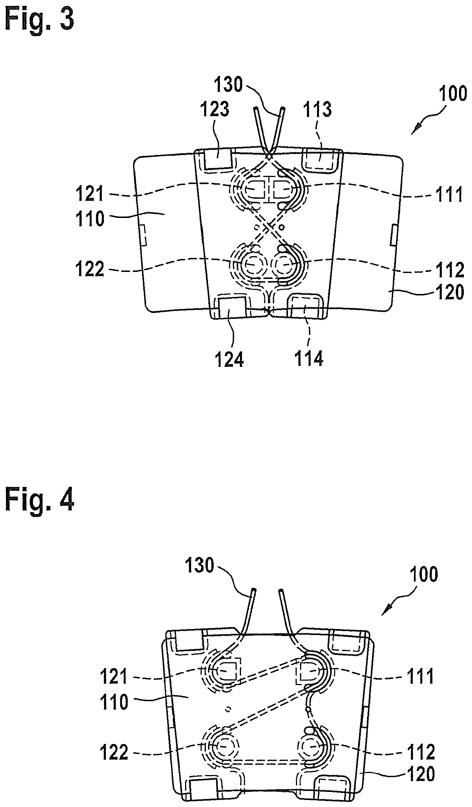

Figur 3 zeigt das Umlenkelement (100) mit den beiden Teilelementen (110, 120) in zusammengeschobener Position, wobei diese Position dadurch erreicht wird, dass die beiden Teilelemente (110, 120) in den randseitigen Führungen (113, 114, 123, 124) des jeweils anderen Teilelements gleiten. In der zusammengeschobenen Position liegen die beiden Seilführungselemente (111, 112) des ersten Teilelements (110) in kleinstmöglicher Entfernung von den beiden Seilführungselementen (121, 122) des zweiten Teilelements (110), so dass die Schnur (130), die abwechselnd von einem Seilführungselement des ersten Teilelements und von einem Seilführungselement des zweiten Teilelements umgelenkt wird auf einem kurzen Weg in dem Umlenkelement (100) verläuft, so dass dabei wenig Schnur verbraucht wird und die Schnur, wenn sie an ihren nicht gezeigten Enden an einem Widerlager befestigt ist, nicht mehr gespannt ist. In der Ausführungsform des Umlenkelements (100) nach den Figuren 1 bis 3 kann sowohl vorgesehen sein, dass ein Verschieben der beiden Teilelemente (110, 120) gegeneinander zum Spannen der Schnur (130) führt als auch vorgesehen sein, dass ein Spannen der Schnur (130) zum Verschieben der beiden Teilelemente (110, 120) gegeneinander führt.

Wie auch in den Figuren 1 bis 3 gezeigt, ist auch in den Figuren 4 bis 6 vorgesehen, dass die Schnur erst durch das erste Seilführungselement des einen Teilelements umgelenkt wird, dann durch das erste Seilführungselement des anderen Teilelements umgelenkt wird, dann durch das zweite Seilführungselement des einen Teilelements umgelenkt wird und dann durch das zweite Seilführungselement des anderen Teilelements umgelenkt wird.

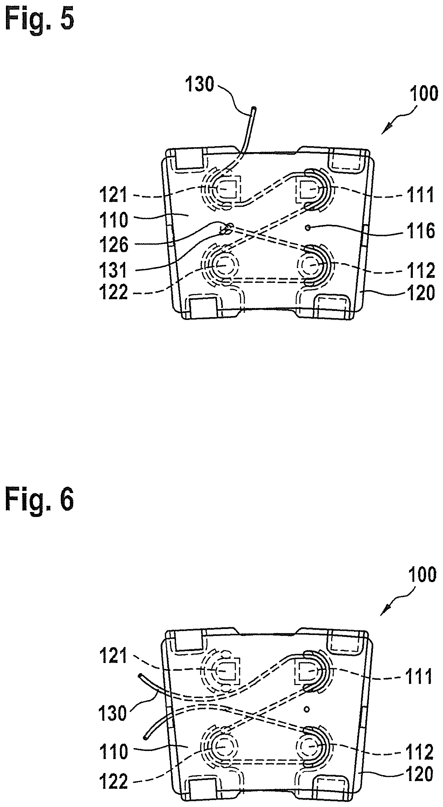

Die Figuren 4 bis 6 zeigen alternative Ausführungsformen des Umlenkelements (100), wobei der Aufbau des Umlenkelements (100) gleich wie in den Figuren 1 bis 3 ist, also auch die beiden Teilelemente (110, 120) mit den Seilführungselemente (111, 112, 121, 122) vorhanden sind, aber die Führung der Schnur (130) eine jeweils andere ist. Während in den Figuren 4 und 6 die Schnur (130) in das Umlenkelement (100) eintritt und nach der Umlenkung wieder aus dem Umlenkelement (100) austritt, ist in Figur 5 ein Ende (131) an einem der Teilelemente, hier das zweite Teilelement (120) befestigt. Daher weisen die plattenförmigen Teilelemente (110,120) auch zusätzliche Löcher (116, 126) auf, durch die wahlweise ebenfalls die Schnur (130) in das Umlenkelement (100) ein- oder austreten kann.

Durch die unterschiedlichen Schnurführungsmöglichkeiten kann in vorteilhafter Weise ein geeigneter Krafteinleitungswinkel gewählt werden.

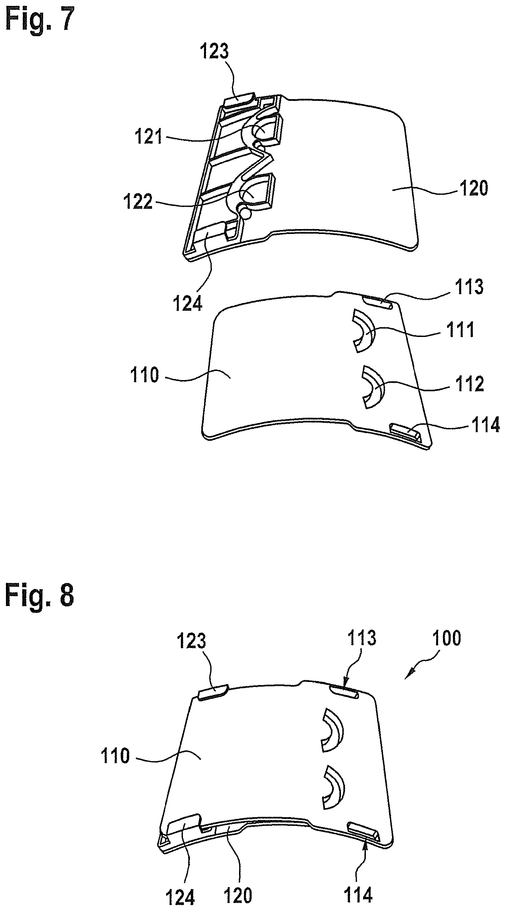

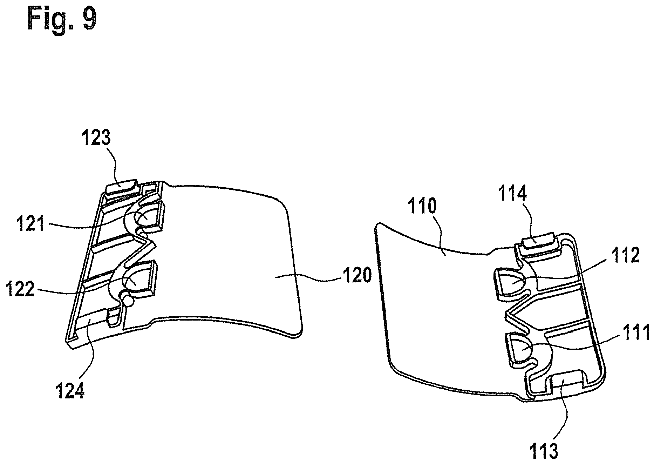

Die Figuren 7 bis 9 zeigen eine weitere Ausführungsform des erfindungsgemäßen Umlenkelements (100) mit den Teilelementen (110, 120), die wieder Seilführungselemente (111, 112, 121, 122) und randseitige Führungen (113, 114, 123, 124) aufweisen. Während in den Figuren 7 und 9 die Teilelemente (110, 120) als Einzelteile gezeigt sind, ist in Figur 8 das zusammengesetzte Umlenkelement (100) zu sehen, jedoch ohne Schnur. Der Schnurverlauf kann bevorzugt wie in den Figuren 1 bis 3, alternativ aber auch anders, beispielsweise wie in den Figuren 4 bis 6 erfolgen. In der gezeigten Ausführungsform findet die Verschiebung der beiden Teilelemente (110, 120) zueinander in einem Kreisbogen statt, so dass durch die Verschiebung die Bogenlänge eines Kreissegmentes verändert wird. Ein solches Umlenkelement kann in vorteilhafter Weise an einer Extremität, beispielsweise an einem Arm, verwendet werden, wobei der Radius des Kreissegments in etwa die Dimension des Radius der Extremität hat. Eine solche Ausführungsform wird auch in der in den Figuren 10 bis 12 gezeigten Orthese zur Dämpfung oder Begrenzung der Gelenkbewegung eines Ellenbogens verwendet.

Bevorzugt kann das Umlenkelement also in einer "Werferorthese" eingesetzt werden, also einer Armorthese, die die Streckung des Arms beim Wurf beispielsweise eines Balls begrenzen soll, sodass es nicht zu einer Überstreckung des Wurfarms kommt. Eine solche ist in den Figuren 10 bis 12 gezeigt. Durch die Vollführung der Streckbewegung des Wurfarms wird der Umfang der Manschette über das Seil verkleinert. Je weiter die Streckung vollendet wird, desto härter packt die Manschette zu und verhindert so die vollständige Armstreckung und gleichsam ihr eigenes Hinaufrutschen am Unterarm. Dabei ermöglicht das erfindungsgemäße Umlenkelement (100), dass die entsprechend starke Kraft des Seils sich flächig verteilen kann, um sich nicht und das Seil nicht in den Unterarm einschneidet.

Die Orthese (200) aus den Figuren 10 bis 12 dient also der Dämpfung oder Begrenzung der Gelenkbewegung eines Arms, und weist eine den Unterarm umgreifende erste Manschette (201) auf, die mit einem am Oberarm anlegbaren Widerlager gekoppelt ist, das als zweite Manschette (202) ausgeführt ist. Von der ersten Manschette (201) erstreckt sich zu der zweiten Manschette (202) ein zweifach und überkreuz geführtes Zugseil (130), das mit den beiden Manschetten kraftschlüssig verbunden ist, wobei das Zugseil über das erfindungsgemäße Umlenkelement (100) mit der ersten Manschette (201) verbunden ist. Somit ist das Umlenkelement (100) als Druckeinleitungsabschnitt der ersten Manschette (201) ausgebildet, wobei das Zugseil (130) derart ausgestaltet ist, dass es im angelegten Zustand der Orthese (200) durch die Gelenkbewegung des Ellenbogens spannbar ist, um dabei über das Umlenkelement (100) eine Kompression auf einen darunter liegenden Weichteilbereich des Unterarms auszuüben, um die Ellenbogenbewegung zu dämpfen oder zu begrenzen. Die Manschetten (201, 202) sind auf ein textiles Gestrick (210) aufgerüstet.

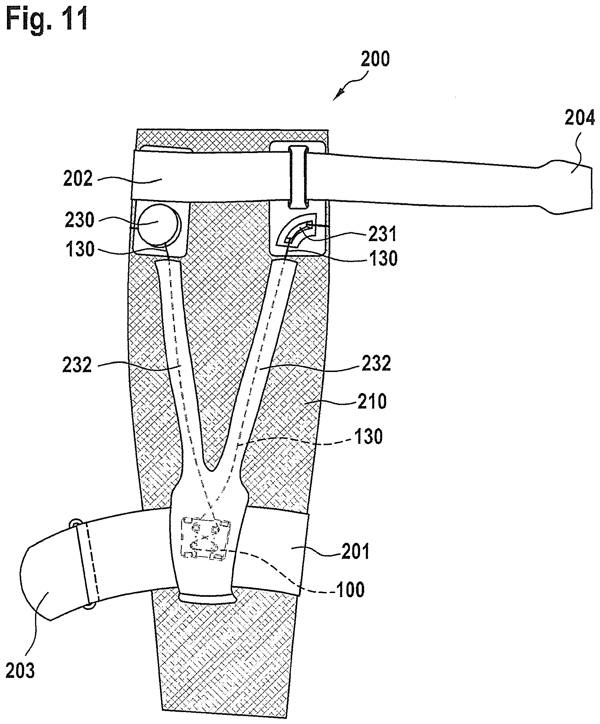

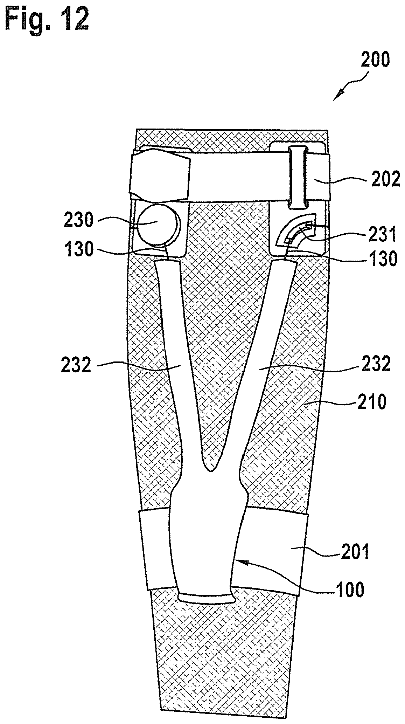

Das Zugseil (130) kann im Bereich der zweiten Manschette (202) durch ein Aufrollelement (230) gespannt werden. Dabei sind beide Enden des Zugseils (130) mit dem Aufrollelement (230) verbunden und werden beim Drehen des Aufrollelements (230) gespannt oder gelöst. Zur Führung des Zugseils (130) zum Aufrollelement (230) können weitere einfache Umlenkelemente (231) aus dem Stand der Technik verwendet werden. Auch kann das Zugseil (130) in Seilkanälen (232) verlaufen, die beispielsweise auf dem textilen Gestrick (210) aufgeschweißt, aufgenäht oder aufgeklebt sind. Die Seilkanäle (232) verdecken in der Figur 12 das Zugseil (130) und das Umlenkelement (100). In den Figuren 10 und 11 sind daher das Umlenkelement (100) und das Zugseil (130) als Strichlinie gezeichnet.

Die Manschetten (201, 202) sind reversibel fixierbar, indem sie mit einem Klettverschluss (203, 204) versehen sind. In Figur 11 sind die geöffneten Klettverschlüsse zu sehen, in den Figuren 10 und 12 sind die Klettverschlüsse geschlossen.

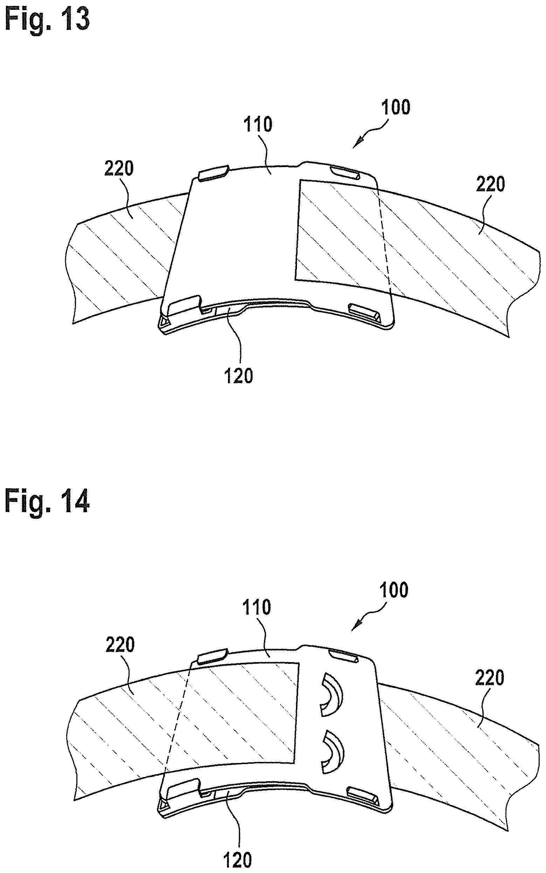

Die Figuren 13 und 14 zeigen das gewölbte Umlenkelement (100) aus Figur 8. An den beiden Teilelementen (110, 120) ist jeweils ein Gurt (220) angebracht, der beispielsweise eine Extremität umfassen kann.

In Figur 13 ist der Gurt (220) auf den Teilelementen (110, 120) so befestigt, dass er beim Zusammenschieben der Teilelemente (110, 120) zusammengezogen wird und somit der Gurtumfang verkleinert wird. In Figur 14 ist der Gurt (220) auf den Teilelementen (110, 120) so befestigt, dass er beim Zusammenschieben der Teilelemente (110, 120) auseinandergezogen wird und somit der Gurtumfang vergrößert wird. Das Zusammenschieben der Teilelemente (110,120) kann insbesondere durch ein Spannen eines nicht gezeigten Spannseils erfolgen, beispielsweise infolge einer Bewegung des Extremitätengelenks der von dem Gurt (220) umfassten Extremität.

Somit kann durch die Wahl der Positionierung eines Gurts auf dem erfindungsgemäßen Umlenkelements in einfacher Weise der gewünschte Effekt beim Zusammenschieben der Teilelemente erreicht werden, also eine Verkleinerung des Gurtumfangs, was zu einem strafferen Anliegen des Gurts an der Extremität führt, oder eine Vergrößerung des Gurtumfangs, was zu einem lockereren Anliegen des Gurts an der Extremität führt.

Alternativ zu einem durchgängigen Gurt können die Teilelemente auch jeweils mit unterschiedlichen Gurten verbunden sein, die beispielsweise auf einem Grundgestrick oder einem anderen Ortheseelement enden.

## Patentansprüche

1. Umlenkelement (100) zum Umlenken eines Seils (130) in orthopädischen oder medizinischen Hilfsmitteln oder Sporthilfsmitteln, wobei das Umlenkelement wenigstens ein erstes Teilelement (110) und ein zweites Teilelement (120) umfasst, wobei das erste Teilelement (110) und das zweite Teilelement (120) mit nur jeweils einem Freiheitsgrad gegeneinander verschiebbar sind, und wobei das erste Teilelement (110) und das zweite Teilelement (120) jeweils mindestens ein Seilführungselement (111, 112, 121, 122) zum Umlenken des Seils (130) aufweisen **dadurch gekennzeichnet, dass** das erste Teilelement (110) und/oder das zweite Teilelement (120) mindestens zwei randseitige Führungen (113, 114, 123, 124) aufweist, in der das jeweils andere Teilelement gleiten kann.

2. Umlenkelement nach Anspruch 1, wobei das Umlenkelement eine Fixiereinrichtung (150) aufweist, durch die der jeweils eine Freiheitsgrad blockiert werden kann.

3. Umlenkelement nach einem der vorhergehenden Ansprüche, wobei die beiden Teilelemente (110, 120) jeweils einen plattenförmigen Grundkörper haben, aus dem auf einer Seite das mindestens eine Seilführungselement (111, 112, 121, 122) und die mindestens zwei randseitigen Führungen (113, 114, 123, 124) herausragen.

4. Umlenkelement nach einem der vorhergehenden Ansprüche, wobei die beiden Teilelemente (110, 120) einen Innenraum bilden, dessen Größe durch das Verschieben der beiden Teilelemente gegeneinander veränderbar ist und die Seilführungselemente (111, 112, 121, 122) in den Innenraum hineinragen.

5. Umlenkelement nach einem der vorhergehenden Ansprüche, wobei die Verschiebung der beiden Teilelemente (110, 120) zueinander in einem Kreisbogen stattfindet und durch die Verschiebung die Bogenlänge eines Kreissegmentes verändert wird.

6. Umlenkelement nach einem der vorhergehenden Ansprüche, umfassend ein Seil (130).

7. Umlenkelement nach Anspruch 6, wobei das erste Teilelement (110) und das zweite Teilelement (120) jeweils zwei Seilführungselemente (111, 112, 121, 122) zum Umlenken des Seils aufweisen und wobei das Seil erst durch das erste Seilführungselement des ersten Teilelements umgelenkt wird, dann durch das erste Seilführungselement des zweiten Teilelements umgelenkt wird, dann durch das zweite Seilführungselement des ersten Teilelements umgelenkt wird und dann durch das zweite Seilführungselement des zweiten Teilelements umgelenkt wird.

8. Umlenkelement nach einem der vorhergehenden Ansprüche, wobei ein Verschieben der beiden Teilelemente (110,120) gegeneinander zum Spannen des Seils (130) führt und/oder wobei ein Spannen des Seils (130) zum Verschieben der beiden Teilelemente (110,120) gegeneinander führt.

9. Umlenkelement nach einem der vorhergehenden Ansprüche, wobei das Seil (130) in das Umlenkelement (100) eintritt und nach der Umlenkung wieder aus dem Umlenkelement (100) austritt oder wobei das Seil (130) in das Umlenkelement (100) eintritt und nach der Umlenkung mit einem Ende am Umlenkelement (100) befestigt ist.

10. Orthopädisches Hilfsmittel, medizinisches Hilfsmittel oder Sporthilfsmittel, umfassend ein Umlenkelement (100) nach einem der vorhergehenden Ansprüche.

11. Orthopädisches Hilfsmittel, medizinisches Hilfsmittel oder Sporthilfsmittel nach Anspruch 10, wobei das orthopädische Hilfsmittel, das medizinische Hilfsmittel oder das Sporthilfsmittel ein Spannseil (130) aufweist, dem das Umlenkelement (100) zugeordnet ist.

12. Orthopädisches Hilfsmittel nach Anspruch 10 oder nach Anspruch 11, wobei das orthopädische Hilfsmittel eine Orthese (200) ist.

13. Orthese nach Anspruch 12, wobei die Orthese (200) eine Orthese zur Dämpfung oder Begrenzung der Gelenkbewegung eines Extremitätengelenks ist, aufweisend eine die Extremität unterhalb des Gelenks umgreifende Manschette (201), die mit einem an der Extremität oberhalb des Gelenks anlegbaren Widerlager (202) gekoppelt ist, wobei sich von dem Widerlager (202) zu der Manschette (201) mindestens ein Zugseil (130) erstreckt, das mit Widerlager (202) und Manschette (201) kraftschlüssig verbunden ist, wobei das Zugseil (130) über das Umlenkelement (100) nach einem der Ansprüche 1 bis 9 mit der Manschette (201) verbunden ist und das Umlenkelement (100) als Druckeinleitungsabschnitt der Manschette (201) ausgebildet ist, und wobei das Zugseil (130) derart ausgestaltet ist, dass es im angelegten Zustand der Orthese (200) durch die Gelenkbewegung der Extremität spannbar ist, um dabei über das Umlenkelement (100) Kompression auf einen darunter liegenden Weichteilbereich der Extremität auszuüben, um die Gelenkbewegung zu dämpfen oder zu begrenzen.

14. Orthese nach Anspruch 13, wobei das Zugseil (130) zweifach und überkreuzt von dem Widerlager (202) zu dem Druckeinleitungsabschnitt der Manschette (201) verläuft und an dem Druckeinleitungsabschnitt durch das Umlenkelement (100) umgelenkt wird.

15. Orthese nach einem der Ansprüche 13 oder 14, wobei das Zugseil (130) im Bereich des Widerlagers (202) durch ein Aufrollelement (230) gespannt werden kann.

## Claims

1. Deflector (100) to deflect a rope (130) in orthopedic or medical accessories, or athletic accessories, wherein the deflector comprises at least one first subcomponent (110) and one second subcomponent (120), wherein the first subcomponent (110) and the second subcomponent (120) are shiftable with only one degree of freedom against one another, respectively, and wherein the first subcomponent (110) and the second subcomponent (120) have at least one rope guidance element (111, 112, 121, 122) to deflect the rope (130), respectively, **characterized in that** the first subcomponent (110) and/or the second subcomponent (120) has/have at least two edge-side guides (113, 114, 123, 124) in which the other subcomponent can slide, respectively.

2. Deflector according to claim 1, wherein the deflector has a fixating device (150), through which the respective one degree of freedom can be blocked.

3. Deflector according to anyone of the preceding claims, wherein both subcomponents (110, 120) have a plate-shaped base body, respectively, from which protrudes the at least one rope guidance element (111, 112, 121, 122) and the at least two edge-side guides (113, 114, 123, 124) on one side.

4. Deflector according to anyone of the preceding claims, wherein both subcomponents (110, 120) form an interior space, whose size is adjustable by means of shifting both subcomponents against one another, and the rope guidance elements (111, 112, 121, 122) protrude into the interior space.

5. Deflector according to anyone of the preceding claims, wherein the shift of both subcomponents (110, 120) towards each other occurs in a circular arc and the shift changes the arc length of a circular segment.

6. Deflector according to anyone of the preceding claims, comprising a rope (130).

7. Deflector according to claim 6, wherein the first subcomponent (110) and the second subcomponent (120) have, respectively, two rope guidance elements (111, 112, 121, 122) to deflect the rope and wherein the rope is first deflected through the first rope guidance element of the first subcomponent, then through the first rope guidance element of the second subcomponent, and then deflected through the second rope guidance element of the first subcomponent, and then deflected through the second rope guidance element of the second subcomponent.

8. Deflector according to anyone of the preceding claims, wherein shifting both subcomponents (110, 120) against one another will lead to tensing the rope (130) and/or wherein tensing the rope (130) will result in a shift of both subcomponents (110, 120) against one another.

9. Deflector according to anyone of the preceding claims, wherein the rope (130) enters into the deflector (100) and exits the deflector (100) after deflection or wherein the rope (130) enters into the deflector (100) and is attached with one end at the deflector (100) after deflection.

10. Orthopedic accessory, medical accessory, or athletic accessory comprising a deflector (100) according to anyone of the preceding claims.

11. Orthopedic accessory, medical accessory, or athletic accessory according to claim 10, wherein the orthopedic accessory, the medical accessory, or the athletic accessory has a tension rope (130) to which the deflector (100) is assigned.

12. Orthopedic accessory according to claim 10 or according to claim 11, wherein the orthopedic accessory is an orthosis (200).

13. Orthosis according to claim 12, wherein the orthosis (200) is an orthosis to dampen or limit the joint movement of a joint of the extremities, having a sleeve (201) encompassing the extremity below the joint, which is coupled with an abutment surface (202) that can be applied above the extremity, wherein at least one pull rope (130) extends from the abutment surface (202) to the sleeve (201), which is connected in a force-fitting manner with abutment surface (202) and sleeve (201), wherein the pull rope (130) is connected via the deflector (100) according to anyone of the claims 1 to 9 with the sleeve (201), and wherein the deflector (100) is formed as pressure introduction section of the sleeve (201), and wherein the pull rope (130) is arranged in such a fashion that the orthosis (200) can be tensed in its applied condition by means of the joint movement of the extremity to thus exercise a compression via the deflector (100) onto the underlying soft tissue area of the extremity in order to dampen or limit any joint movements.

14. Orthosis according to claim 13, wherein pull the rope (130) progresses twofoldly and is crossed over by the abutment surface (202) to the pressure introduction section of sleeve (201), and is deflected at the pressure introduction section by means of the deflector (100).

15. Orthosis according to anyone of the claims 13 or 14, wherein the pull rope (130) in the area of the abutment surface (202) can be tensed by means of a roll-up element (230).

## Revendications

1. Élément de renvoi (100) pour renvoyer un câble (130) dans des aides orthopédique, des aides médicales ou des aides de sport, dans lequel l'élément de renvoi comprend au moins un premier élément partiel (110) et un second élément partiel (120), dans lequel le premier élément partiel (110) et le second élément partiel (120) sont déplaçables l'un contre l'autre respectivement avec un seul degré de liberté, et dans lequel le premier élément partiel (110) et le second élément partiel (120) chacun présentent au moins un élément de guidage de câble (111, 112, 121, 122) pour dévier le câble (130), **caractérisé en ce que** le premier élément partiel (110) et/ou le second élément partiel (120) présente(nt) au moins deux guides de bord (113, 114, 123, 124) dans lesquels l'autre élément partiel respectif peut glisser.

2. Élément de renvoi selon la revendication 1, dans lequel l'élément de renvoi présente un dispositif de fixation (150) par lequel le degré de liberté respectif peut être bloqué.

3. Élément de renvoi selon l'une quelconque des revendications précédentes, dans lequel les deux éléments partiels (110, 120) chacun présentent un corps de base sous forme de plaque duquel font saillie, sur un côté, l'au moins un élément de guidage de câble (111, 112, 121, 122) et les au moins deux guides de bord (113, 114, 123, 124).

4. Élément de renvoi selon l'une quelconque des revendications précédentes, dans lequel les deux éléments partiels (110, 120) forment un espace intérieur dont la taille peut être changée par le déplacement des deux éléments partiels l'un contre l'autre, et les éléments de guidage de câble (111, 112, 121, 122) font saillie dans l'espace intérieur.

5. Élément de renvoi selon l'une quelconque des revendications précédentes, dans lequel le déplacement des deux éléments partiels (110, 120) l'un vers l'autre s'effectue dans un arc de cercle et la longueur d'arc d'un segment de cercle est changée par ce déplacement.

6. Élément de renvoi selon l'une quelconque des revendications précédentes, l'élément comprenant un câble (130).

7. Élément de renvoi selon la revendication 6, dans lequel le premier élément partiel (110) et le second élément partiel (120) chacun présentent deux éléments de guidage de câble (111, 112, 121, 122) pour renvoyer le câble, et dans lequel le câble est renvoyé d'abord par le premier élément de guidage de câble du premier élément partiel, puis par le premier élément de guidage de câble du second élément partiel, puis par le second élément de guidage de câble du premier élément partiel, et puis par le second élément de guidage de câble du second élément partiel.

8. Élément de renvoi selon l'une quelconque des revendications précédentes, dans lequel un déplacement des deux éléments partiels (110, 120) l'un contre l'autre effectue une tension du câble (130) et/ou dans lequel une tension du câble (130) effectue un déplacement des deux éléments partiels (110, 120) l'un contre l'autre.

9. Élément de renvoi selon l'une quelconque des revendications précédentes, dans lequel le câble (130) entre dans l'élément de renvoi (100) et puis sorte de l'élément de renvoi (100) après étant renvoyé, ou dans lequel le câble (130) entre dans l'élément de renvoi (100) et, après étant renvoyé, est fixé sur l'élément de renvoi (100) avec une extrémité.

10. Aide orthopédique, aide médical ou aide de sport comprenant un élément de renvoi (100) selon l'une quelconque des revendications précédentes.

11. Aide orthopédique, aide médical ou aide de sport selon la revendication 10, dans lequel l'aide orthopédique, l'aide médical ou l'aide de sport présente un câble de tension (130) auquel est associé l'élément de renvoi (100).

12. Aide orthopédique selon la revendication 10 ou selon la revendication 11, dans lequel l'aide orthopédique est une orthèse (200).

13. Orthèse selon la revendication 12, l'orthèse (200) est un orthèse pour amortir ou limiter le mouvement articulaire d'une articulation d'un membre, et ayant un manchon (201) entourant le membre au-dessous de l'articulation, le manchon étant couplé avec une butée (202) applicable au membre au-dessus de l'articulation, dans laquelle au moins un câble de traction (130) qui est relié de manière frictionnelle avec la butée (202) et le manchon (201) s'étend à partir de la butée (202) jusqu'au manchon (201), dans laquelle le câble de traction (130) est relié au manchon (201) au moyen de l'élément de renvoi (100) selon l'une quelconque des revendications 1 à 9, et l'élément de renvoi (100) est configuré en tant que portion d'introduction de pression du manchon (201), et dans laquelle le câble de traction (130) est configuré de telle manière qu'il peut être tendu, dans l'état appliqué de l'orthèse (200), par le mouvement articulaire du membre, afin d'exercer, au moyen de l'élément de renvoi (100), une compression sur une région de tissu mou sous-jacente du membre pour amortir ou limiter le mouvement articulaire.

14. Orthèse selon la revendication 13, dans laquelle le câble de traction (130) s'étend deux fois et de manière croisée à partir de la butée (202) jusqu'à la portion d'introduction de pression du manchon (201), et est renvoyé au niveau de la portion d'introduction de pression par l'élément de renvoi (100).

15. Orthèse selon l'une quelconque des revendications 13 ou 14, dans laquelle le câble de traction (130) peut être tendu au niveau de la butée (202) par un élément enrouleur (230).
